# EUROPEAN PATENT SPECIFICATION

(11) **EP 3 074 204 B1**
(45) Date of publication and mention of the grant of the patent: **27.12.2017**
(21) Application number: 14802875.6
(22) Date of filing: 24.11.2014
(51) Int. Cl.: B29C 65/16, B01L 3/00, B81C 3/00

(54) **A METHOD FOR LASER WELDING A DISPOSABLE TEST-UNIT**
VERFAHREN ZUM LASERSCHWEISSEN EINER WEGWERF-TESTEINHEIT
PROCÉDÉ DE SOUDAGE AU LASER D'UNE UNITÉ DE TEST JETABLE

(30) Priority: 27.11.2013 EP 13194706
(43) Date of publication of application: 05.10.2016
(73) Proprietor: Roche Diabetes Care GmbH, 68305 Mannheim (DE); F. Hoffmann-La Roche AG, 4070 Basel (CH)
(72) Inventor: BABIC, Branislav, 67071 Ludwigshafen (DE); HORN, Carina, 68647 Biblis (DE)
(74) Representative: Pfiz, Thomas
(86) International application number: PCT/EP2014/075429
(87) International publication number: WO 2015/078821

(56) References cited:
- EP-A1- 1 864 784
- US-A1- 2007 278 097

## Description

The invention concerns a method for laser welding a disposable test-unit for analyzing a body-fluid comprising the steps of providing in a stacked manner a base element, a cover element and optionally an intermediate element, wherein one of said elements is an absorbing element configured to absorb radiation of a laser beam and at least one other of said elements is a transparent element which is permeable for the laser radiation, directing a laser beam in a weld area through the at least one transparent element and against the absorbing element, and fusing together the transparent and the absorbing element to form a test-unit configured to receive body-fluid. The invention further concerns a disposable test-unit manufactured by such a method.

In designs of diagnostic test strips it is known to combine several layers by means of double sided adhesive tapes, which allows processing from roll-to-roll of web material thus achieving a high output and yield in the manufacturing process. However, the use of adhesive tapes contributes to the production costs and often requires specific adhesive compounds adapted to the chemistry of the test.

It is also known from the European Patent Application EP 1 864 784 A1 to fabricate multilayer biosensors by means of laser welding, wherein a laser-transparent material is fused on a laser-absorbing material. The latter is melted by the laser energy and connected to the transparent material. To date, such techniques were limited to clean "black" and "clear" layer configurations.

On this basis the object of the invention is to further improve the known methods and products and to achieve an improved material and production efficiency and a reliable test architecture.

The combination of features stated in the independent claim 1 or 15 is proposed to achieve this object. Advantageous embodiments and further developments of the invention are derived from the dependent claims.

The invention is based on the idea of processing coated assemblies for allowing specific sample interaction in a specific test format. Correspondingly it is proposed according to the invention providing one of said elements in the initial step with a coating in the form of a dry chemistry layer which is adapted to react with an analyte in the body fluid when conducting a test, wherein said coating covers the weld area at least in part and absorbs and/or scatters the laser radiation at least in part. The laser welding technology obviates the need for adhesives and adhesive tapes, reduces the material expenditure and avoids additional process steps. Surprisingly it has been found that coated structures, which absorb and/or scatter at least a part of the utilized laser radiation, do not impede or weaken the resulting connection with regard to weld strength and sealing ability. Breaking down a prejudice among experts, it has been found that such laser responsive interfaces even contribute to the formation of an adherent composite. Moreover, specific interaction with the sample fluid can be integrated in the test architecture.

In a preferred embodiment, the coating may contain one or more components that melt upon impact of the laser beam.

In order to further improve fluidic interactions for the test purposes, it is advantageous when the coating is adapted to increase the wettability of the coated element when wetted with the body fluid.

In this context, it is also advantageous when the coating comprises a detergent and/or a hydrophilic component.

In another embodiment specifically intended for optical measurements, it is favorable when the coating contains light-scattering particles, specifically pigments for example consisting of TiO2, BaTiO3, ZrO2, ZrSiO3 and/or BaSO4.

A further improvement in this direction can be achieved when the coating comprises at least one of organic polymers, pigments, mineral fillers.

For a simplified manufacturing process it is advantageous when the coating is deposited as a chemistry layer on a foil blank to form the intermediate element, and when the intermediate element is laser welded in-between the base element and the cover element.

A further improvement in the test architecture can be achieved by forming a capillary channel configured for transport of body fluid in the base element and/or in the cover element, and by arranging the coating at least in part in the area of the capillary channel.

Advantageously, the base element and the cover element are fused together in a 1-dimensional connection along a continuous or intermittent line.

Another particularly advantageous embodiment provides that the weld area is formed as a weld seam that seals a zone of the test-unit configured to receive body-fluid.

For a further reduction of the constructional expenditure it is advantageous when the base and cover elements are cut from a foil material.

It is also conceivable that continuous webs as feed material for the base and cover elements are transported from roll-to-roll, and that the continuous webs are laser welded to form multiple test units.

In a specific advantageous embodiment, the test unit is formed as a test strip that can be manually handled or as a test tape that can be wound on a spool, e.g. in a tape cassette.

Advantageously, the base element and the cover element are cut as blanks from a foil material, wherein one foil material is generally laser beam absorbent and the other foil material is generally transparent for a welding laser-beam.

In another specific embodiment intended for highly integrated tests, the base and cover elements are molded as a 3D-formed part from a plastic material.

In order to strengthen the resulting weld, it is advantageous when said fusing step includes simultaneous pressing of said constructional elements to form a composite member.

The invention also concerns a disposable test-unit for analyzing a body-fluid comprising a stack of a base element, a cover element and optionally an intermediate element, wherein one of said elements is made of an absorbing material configured to absorb radiation of a laser beam and at least one other of said elements is made of a transparent material that is permeable for a laser radiation, the absorbing and transparent materials being fused together in a weld area by laser welding seams, wherein at least one of said elements is provided with a coating in the form of a dry chemistry layer which is adapted to react with an analyte in the body fluid when conducting a test, wherein the coating covers the weld area at least in part and contains one or more components that absorb and/or scatter said laser radiation at least in part.

It shall be understood that all of the above detailed method aspects relate to the disposable test-unit produced by such method in an analogous way.

The invention is further elucidated in the following on the basis of embodiment examples shown schematically in the drawings, where
- Fig. 1: is a perspective view of a test-unit or capillary test strip consisting of sheet material fused by laser welding;
- Fig. 2: is an exploded, perspective view illustrating an alternate including an intermediate element of fusible material;
- Fig. 3: shows a top view of a portion of another alternate embodiment including a molded base element and a reagent strip;
- Fig. 4: shows the assembled embodiment of fig. 3 including a laser fused cover element;
- Fig. 5: is a diagrammatic view of a laser welding system for producing composite test-units.

Referring to the drawings, a composite test unit 10 as a disposable for a diagnostic test can be prepared by fusing of multiple layers or elements along laser weld lines 12, thereby avoiding the use of adhesive components.

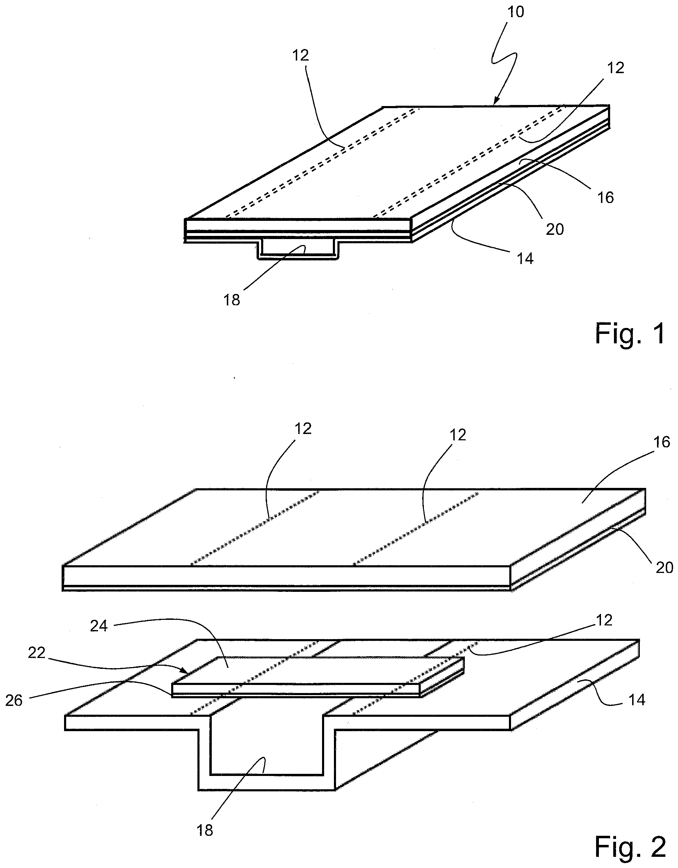

In the embodiment illustrated in fig. 1, a base element 14 consists of a fusible layer which absorbs laser radiation, whereas a transparent cover element 16 consists of a layer which is permeable for the laser radiation. The base element 14 comprises an absorptive (or black) foil blank with a deep-drawn capillary channel 18. The channel 18 can be loaded with a body-fluid sample e.g. for a blood glucose test. On the side facing the channel 18, the flat cover element 16 is provided with a coating 20 which is adapted to interact with the body fluid when conducting a test.

The coating 20 may be configured to promote the wettability or hydrophilic properties of the coated area, such to promote the transport or distribution of the body fluid. For instance, the cover element 16 may be formed from a transparent polycarbonate foil of e.g. 140 µm thickness, and the coating 20 may comprise polar coating agents such as hydroxyethyl cellulose (e.g. available under trademark Tylose from SE Tylose GmBH & Co. KG) and colloidal silica (e.g. available under trademark Bindzil from Akzo Nobel N.V.).

As the coating 20 is applied across the entire interface side of the cover element 16, it also overlaps the weld area, i.e. the lines 12 where the laser beam passes during laser welding. In this process, the laser beam is directed through the transparent cover element 16 and its coating 20 against the base element 14, where the dark material is capable of absorbing laser energy and melting to bond to the adjacent cover element 16. As the weld lines 12 border and seal the channel 18 on both sides, body fluid is prevented from bypassing the transport zone. At the same time, the body fluid receiving area is shielded against environmental influence.

Although the cover element 16 is transmissive for the laser light, the coating 20 contains components which absorb and/or scatter laser radiation at least in part. These components may also melt upon impact of the laser beam. Surprisingly, it has been found that such shading or scattering does not affect the strength and seal of the weld lines 12. In this context, it should be understood that the weld lines 12 are arranged at a distance from the edges of the test unit 10, and the laser energy is adjusted for proper welding, but not for cutting at the same time.

In the embodiment shown in fig. 2, the same or similar parts have been provided with the same reference numerals as previously described. This embodiment differs in that an intermediate reagent element 22 is interposed between the base element 14 and the cover element 16.

The reagent element 22 consists of a transparent foil blank or carrier 24 and a dry chemistry layer 26 deposited on the carrier 24 and overlapping a part of the channel 18. The chemistry layer 26 is adapted to irreversibly react with an analyte, e.g. glucose in the body fluid, such that a product of the reaction can be detected e.g. by a reflection-photometric device. For this purpose, the layer 26 comprises organic polymers, pigments and mineral fillers. The pigments effect an increase in the strength of the measurement signal and may be selected from TiO2, BaTiO3, ZrO2, ZrSiO3 and/or BaSO4. It is also envisioned that fine-grained particles can be contained in the chemistry layer 26 which have a strong light-scattering effect due to a high refractive index of e.g. at least 2,5.

The stack of layered elements 14, 22, 16 is subjected to pressing and simultaneous welding action along weld lines 12, where the laser beam is directed through the cover element 14 and the intermediate element 22 onto the fusible base element 14. Again, surprisingly it has been found that such layer compositions of an intermediate element 22 do not significantly weaken the resulting laser welds 12.

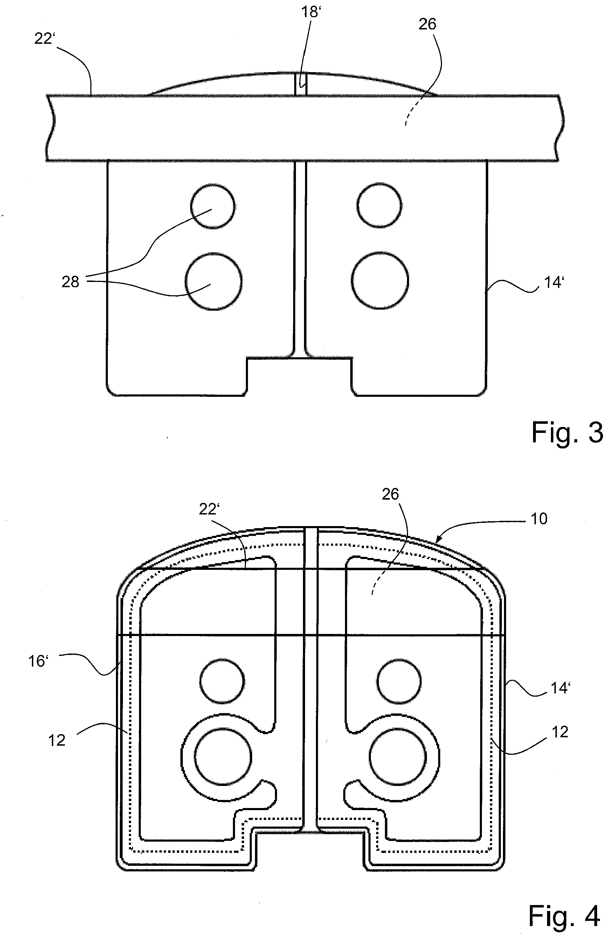

Figs. 3 and 4 illustrate another embodiment of a composite test unit 10 for a single-use diagnostic test. In this example, the laser welded members are molded 3-dimensional plastic elements 14', 16' in combination with an intermediate reagent strip 22'. The unit 10 is intended for a one step test, where an integrated needle (not shown) is used in a reciprocating movement to pierce the skin of a user and to apply sampled blood onto the test strip 22'.

As best seen from fig. 3, the base element 14' has a channel 18' provided to guide an inserted sampling needle. Further, hole structures 28 allow form-fitting connection to a measuring device which also has a drive to engage the needle. The base element 14' is absorptive with respect to laser radiation used for welding. Furthermore, the test strip 22' comprises a chemistry layer or coating 26 facing the channel 18' and being adapted to react with an analyte in the body fluid, wherein the coating 26 absorbs and/or scatters the laser radiation at least in part.

Fig. 4 shows the assembled composite unit 10 including a transparent cover element 16' which is fused to the base element 14' by laser welding along peripheral weld lines 12. As in the embodiments exemplified above, the coating 26 covers the weld area at least in part and hence takes up a fraction of the laser energy. Albeit it has been proved that the laser welded elements 14', 16' can be firmly connected to such an extent that a manual disassembly is not possible.

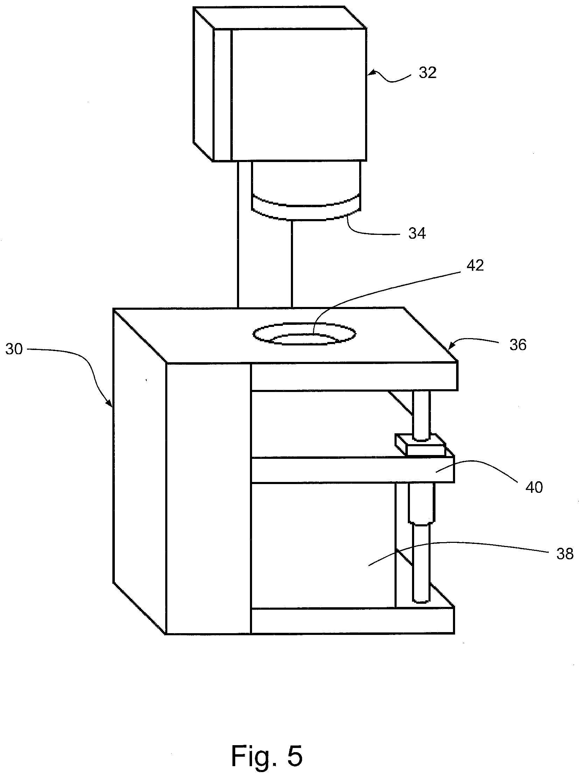

Fig. 5 illustrates a laser welding system 30 useful in forming the composite test units 10 of the present invention. The system 30 comprises a laser apparatus 32 including optics 34, a clamping unit 36 for the components to be welded and a hydraulic actuator 38 to actuate the clamping unit 36. The latter includes a carrier plate 40 to position the elements 14, 16, 22 in a stacked manner and a transparent counter plate 42, The carrier plate 40 can be moved upwards by means of the hydraulic actuator 38, such that pressure is put on the composite layers during activation of the laser apparatus 32. In the welding process, the coating layers 20, 26 melt upon laser impact and are fused together with the base and cover elements 14, 16.

## Claims

1. A method for laser welding a disposable test-unit (10) comprising
a) providing in a stacked manner a base element (14), a cover element (16) and optionally an intermediate element (22), wherein one of said elements is an absorbing element configured to absorb radiation of a laser beam and at least one other of said elements is a transparent element which is permeable for the laser radiation,
b) directing a laser beam in a weld area (12) through the at least one transparent element and against the absorbing element,
c) fusing together the transparent and the absorbing element to form a test-unit (10) configured to receive body-fluid,
d) providing one of said elements (14,16,22) in step a) with a coating (20;26) that covers the weld area (12) at least in part and absorbs and/or scatters the laser radiation at least in part,
**characterized in that**
said coating (20;26) is in the form of a chemistry layer which is adapted to react with an analyte in the body fluid when conducting a test.

2. The method of claim 1, wherein the coating (20;26) contains one or more components that melt upon impact of the laser beam.

3. The method of claim 1 or 2, wherein the coating (20) is adapted to increase the wettability of the coated element (16) when wetted with the body fluid.

4. The method of one of the claims 1 to 3, wherein the coating (26) contains light-scattering particles, specifically pigments for example consisting of TiO2, BaTi03, Zr02, ZrSiO3 and/or BaSO4.

5. The method of one of the claims 1 to 4, wherein the coating (26) comprises at least one of organic polymers, pigments, mineral fillers.

6. The method of one of the claims 1 to 5, further comprising:
depositing the coating (20;26) as a chemistry layer on a foil blank to form the intermediate element (22) and laser welding the intermediate element (22) in-between the base element (14) and the cover element (16).

7. The method of one of the claims 1 to 6, further comprising:
forming a capillary channel (18) configured for transport of body fluid in the base element (14) and/or in the cover element (16), and arranging the coating (20;26) at least in part in the area of the capillary channel (18).

8. The method of one of the claims 1 to 7, wherein the base element (14) and the cover element (16) are fused together along a continuous or intermittent line.

9. The method of one of the claims 1 to 8, wherein the weld area (12) is formed as a weld seam that seals a zone of the test-unit (10) configured to receive body-fluid.

10. The method of one of the claims 1 to 9, further comprising:
transporting from roll-to-roll continuous webs as feed material for the base and cover elements (14,16), laser welding the continuous webs to form multiple test-units (10).

11. The method of one of the claims 1 to 10, wherein the test-unit (10) is formed as a test strip that can be manually handled or a test tape that can be wound on a spool.

12. The method of one of the claims 1 to 11, wherein the base element (14) and the cover element (16) are cut from a foil material which is generally laser-beam absorbent or permeable.

13. The method of one of the claims 1 to 12, further comprising:
molding the base and cover elements (14,16) as 3D-formed parts from a plastic material.

14. The method of one of the claims 1 to 13, wherein said fusing includes simultaneous pressing of said elements (14,16,22) to form a composite member.

15. A disposable test-unit (10) for analyzing a body-fluid comprising a stack of a base element (14), a cover element (16) and optionally an intermediate element (22), wherein one of said elements is made of an absorbing material configured to absorb radiation of a laser beam and at least one other of said elements is made of a transparent material that is permeable for a laser radiation, the absorbing and transparent materials being fused together in a weld area (12) by laser welding seams, wherein at least one of said elements is provided with a coating (20;26) that covers the weld area (12) at least in part and contains one or more components that absorb and/or scatter said laser radiation at least in part,
**characterized in that**
said coating (20;26) is in the form of a chemistry layer which is adapted to react with an analyte in the body fluid when conducting a test.

## Patentansprüche

1. Verfahren zu Laserschweißen einer Einweg-Testeinheit (10) umfassend
a) Bereitstellen eines Basiselements (14), eines Deckelements (16) und gegebenenfalls eines Zwischenelements (22) in einer gestapelten Weise, wobei eines der Elemente ein absorbierendes Element ist, welches dazu ausgelegt ist, Strahlung eines Laserstrahls zu absorbieren, und wenigstens ein anderes der Elemente ein transparentes Element ist, welches für die Laserstrahlung durchlässig ist,
b) Lenken eines Laserstrahls in einer Schweißzone (12) durch das wenigstens eine transparente Element und gegen das absorbierende Element,
c) Zusammenschweißen des transparenten und des absorbierenden Elements, um eine zum Aufnehmen von Körperflüssigkeit ausgelegte Testeinheit (10) zu bilden,
**gekennzeichnet durch**
d) Bereitstellen eines der Elemente (14,16,22) in Schritt a) mit einer Beschichtung (20;26) in Form einer Chemieschicht, die dazu ausgebildet ist, beim Durchführen eines Tests mit einem Analyten in der Körperflüssigkeit zu reagieren, wobei die Beschichtung (20;26) die Schweißzone (12) zumindest teilweise bedeckt und die Laserstrahlung zumindest teilweise absorbiert und/oder streut.

2. Verfahren nach Anspruch 1, bei welchem die Beschichtung (20;26) eine oder mehrere Komponenten enthält, die beim Auftreffen des Laserstrahls schmelzen.

3. Verfahren nach Anspruch 1 oder 2, bei welchem die Beschichtung (20) dazu ausgelegt ist, die Benetzbarkeit des beschichteten Elements zu erhöhen, wenn dieses mit der Körperflüssigkeit benetzt ist.

4. Verfahren nach einem der Ansprüche 1 bis 3, wobei die Beschichtung (26) licht-streuende Partikel enthält, insbesondere Pigmente, beispielsweise bestehend aus Ti02, BaTi03, Zr02, ZrSi03 und/oder BaS04.

5. Verfahren nach einem der Ansprüche 1 bis 4, wobei die Beschichtung (26) wenigstens eines von organischen Polymeren, Pigmenten, mineralischen Füllstoffen enthält.

6. Verfahren nach einem der Ansprüche 1 bis 5, weiterhin umfassend:
Aufbringen der Beschichtung (20;26) als eine Chemieschicht auf einen Folienzuschnitt, um das Zwischenelement (22) zu bilden, und Laserschweißen des Zwischenelements (22) zwischen dem Basiselement (14) und dem Deckelement (16).

7. Verfahren nach einem der Ansprüche 1 bis 6, weiterhin umfassend: Bilden eines Kapillarkanals (18), der für einen Transport von Körperflüssigkeit im dem Basiselement (14) und/oder dem Deckelement (16) ausgelegt ist und Anordnen der Beschichtung (20;26) zumindest teilweise im Bereich des Kapillarkanals (18).

8. Verfahren nach einem der Ansprüche 1 bis 7, bei welchem das Basiselement (14) und das Deckelement (16) entlang einer kontinuierlichen oder unterbrochenen Linien zusammengeschweißt sind.

9. Verfahren nach einem der Ansprüche 1 bis 8, bei welchem die Schweißzone (12) als Schweißnaht gebildet ist, die eine zum Aufnehmen von Körperflüssigkeit ausgebildete Zone der Testeinheit (10) abdichtet.

10. Verfahren nach einem der Ansprüche 1 bis 9, weiterhin umfassend:
Transportieren von Rolle-zu-Rolle eines kontinuierlichen Bands als Ausgangsmaterial für das Basis- und Deck-Element (14,16), Laserschweißen der kontinuierlichen Bänder, um vielfach Test-Einheiten (10) zu bilden.

11. Verfahren nach einem der Ansprüche 1 bis 10, wobei die Testeinheit (10) als ein manuell handhabbarer Teststreifen oder ein auf eine Spule aufwickelbares Testband gebildet ist.

12. Verfahren nach einem der Ansprüche 1 bis 11, bei welchem das Basiselement (14) und das Deckelement (16) aus einem Folienmaterial geschnitten sind, welches allgemein laserstrahlabsorbierend oder -durchlässig ist.

13. Verfahren nach einem der Ansprüche 1 bis 12, weiterhin umfassend:
Formen des Grund- und Deckelements (14,16) als 3-D-Formteile aus einem Plastikmaterial.

14. Verfahren nach einem der Ansprüche 1 bis 13, bei welchem das genannte Schweißen zugleich Zusammenpressen der Elemente (14,16,22) zum Bilden eines Verbundteils umfasst.

15. Einweg-Testeinheit (10) zum Analysieren einer Körperflüssigkeit umfassend einen Stapel aus einem Basiselement (14), einem Deckelement (16) und gegebenenfalls einem Zwischenelement (22), wobei eines der Elemente aus einem absorbierenden Material gebildet ist, welches dazu ausgelegt ist, Strahlung eines Laserstrahls zu absorbieren, und wenigstens ein anderes der Elemente aus einem transparenten Material gebildet ist, das für die Laserstrahlung durchlässig ist, wobei die absorbierenden und transparenten Materialien in einer Schweißzone (12) durch Laserschweißnähte zusammengeschweißt sind, wobei wenigstens eines der Elemente mit einer Beschichtung (20;26) in Form einer Chemieschicht versehen ist, welche dazu ausgelegt ist, beim Durchführen eines Tests mit einem Analyten in der Körperflüssigkeit zu reagieren, wobei die Beschichtung (20;26) die Schweißzone (12) zumindest teilweise bedeckt und eine oder mehrere Komponenten enthält, die zumindest teilweise Laserstrahlung absorbieren und/oder streuen.

## Revendications

1. Procédé destiné à souder au laser une unité de test jetable (10) comprenant les étapes consistant à
a) fournir d'une manière empilée un élément de base (14), un élément de recouvrement (16) et facultativement un élément intermédiaire (22), dans lequel l'un desdits éléments est un élément absorbant configuré pour absorber le rayonnement d'un faisceau laser et au moins un autre desdits éléments est un élément transparent qui est perméable au rayonnement laser,
b) diriger un faisceau laser dans une zone de soudure (12) à travers l'au moins un élément transparent et contre l'élément absorbant,
c) unir par fusion l'élément transparent et l'élément absorbant pour former une unité de test (10) configurée pour recevoir un fluide corporel,
d) doter l'un desdits éléments (14, 16, 22) lors d'une étape a) d'un revêtement (20 ; 26) qui recouvre la zone de soudure (12) au moins en partie et absorbe et/ou diffuse le rayonnement laser au moins en partie,
**caractérisé en ce que**
ledit revêtement (20 ; 26) se présente sous la forme d'une couche chimique qui est adaptée pour réagir avec un analyte dans le fluide corporel lors de la réalisation d'un test.

2. Procédé selon la revendication 1, dans lequel le revêtement (20 ; 26) contient un ou plusieurs composants qui fondent au moment de l'impact du faisceau laser.

3. Procédé selon la revendication 1 ou 2, dans lequel le revêtement (20) est adapté pour augmenter la mouillabilité de l'élément revêtu (16) lorsqu'il est mouillé avec le fluide corporel.

4. Procédé selon l'une des revendications 1 à 3, dans lequel le revêtement (26) contient des particules de diffusion de la lumière, spécifiquement des pigments constitués par exemple de TiO₂, BaTiO₃, ZrO₂, ZrSiO₃ et/ou BaSO₄.

5. Procédé selon l'une des revendications 1 à 4, dans lequel le revêtement (26) comprend au moins l'un parmi des polymères organiques, des pigments, des charges de remplissage minérales.

6. Procédé selon l'une des revendications 1 à 5, comprenant en outre :
déposer le revêtement (20 ; 26) en tant que couche chimique sur une découpe de feuille pour former l'élément intermédiaire (22) et souder au laser l'élément intermédiaire (22) entre l'élément de base (14) et l'élément de recouvrement (16).

7. Procédé selon l'une des revendications 1 à 6, comprenant en outre :
former un canal capillaire (18) configuré pour le transport de fluide corporel dans l'élément de base (14) et/ou dans l'élément de recouvrement (16), et agencer le revêtement (20 ; 26) au moins en partie dans la zone du canal capillaire (18).

8. Procédé selon l'une des revendications 1 à 7, dans lequel l'élément de base (14) et l'élément de recouvrement (16) sont unis par fusion le long d'une ligne continue ou intermittente.

9. Procédé selon l'une des revendications 1 à 8, dans lequel la zone de soudure (12) est formée en tant que joint de soudure qui rend étanche une zone de l'unité de test (10) configurée pour recevoir un fluide corporel.

10. Procédé selon l'une des revendications 1 à 9, comprenant en outre :
transporter de rouleau à rouleau des toiles continues en tant que matériau d'alimentation pour les éléments de base et de recouvrement (14, 16), souder au laser les toiles continues pour former de multiples unités de test (10).

11. Procédé selon l'une des revendications 1 à 10, dans lequel l'unité de test (10) est formée en tant que bande de test qui peut être manipulée manuellement ou un ruban de test qui peut être enroulé sur une bobine.

12. Procédé selon l'une des revendications 1 à 11, dans lequel l'élément de base (14) et l'élément de recouvrement (16) sont découpés à partir d'un matériau en feuille qui, globalement, absorbe le faisceau laser ou est perméable à celui-ci.

13. Procédé selon l'une des revendications 1 à 12, comprenant en outre :
mouler les éléments de base et de recouvrement (14, 16) en tant que pièces formées en 3D à partir d'un matériau en plastique.

14. Procédé selon l'une des revendications 1 à 13, dans lequel ladite fusion comprend une compression simultanée desdits éléments (14, 16, 22) pour former un élément composite.

15. Unité de test jetable (10) destinée à analyser un fluide corporel comprenant un empilement d'un élément de base (14), d'un élément de recouvrement (16) et facultativement d'un élément intermédiaire (22), dans laquelle l'un desdits éléments est constitué d'un matériau absorbant configuré pour absorber le rayonnement d'un faisceau laser et au moins un autre desdits éléments est constitué d'un matériau transparent qui est perméable à un rayonnement laser, les matériaux absorbant et transparent étant unis par fusion dans une zone de soudure (12) par des joints de soudure au laser, dans laquelle au moins l'un desdits éléments est doté d'un revêtement (20 ; 26) qui recouvre la zone de soudure (12) au moins en partie et contient un ou plusieurs composants qui absorbent et/ou diffusent ledit rayonnement laser au moins en partie,
**caractérisée en ce que** ledit revêtement (20 ; 26) se présente sous la forme d'une couche chimique qui est adaptée pour réagir avec un analyte dans le fluide corporel lors de la réalisation d'un test.
